# FASCICULE DE BREVET EUROPEEN

(11) **EP 2 162 428 B1**
(45) Date de publication et mention de la délivrance du brevet: **28.03.2012**
(21) Numéro de dépôt: 08805814.4
(22) Date de dépôt: 20.05.2008
(51) Int. Cl.: C07C 253/22, C07C 209/48, C07C 68/00, C07C 255/03, C07C 255/07, C07C 211/03, C07C 211/21

(54) **PROCÉDÉ DE CO-PRODUCTION DE CARBONATES NON CYCLIQUES ET DE NITRILES ET/OU D'AMINES GRAS**
VERFAHREN ZUR GLEICHZEITIGEN HERSTELLUNG VON NICHTZYKLISCHEN CARBONATEN UND FETTSÄURENITRILEN UND/ODER FETTSÄUREAMINEN
METHOD FOR THE CO-PRODUCTION OF NON-CYCLIC CARBONATES AND FATTY NITRILES AND/OR AMINES

(30) Priorité: 22.05.2007 FR 0755185
(43) Date de publication de la demande: 17.03.2010
(73) Titulaire: ARKEMA FRANCE, 92700 Colombes (FR)
(72) Inventeur: DUBOIS, Jean-Luc, F-69390 Millery (FR); GILLET, Jean-Philippe, F-69530 Brignais (FR)
(86) Numéro de dépôt international: PCT/FR2008/050868
(87) Numéro de publication internationale: WO 2008/145940

(56) Documents cités:
- GB-A- 1 388 053
- US-A- 5 175 370
- US-A- 6 005 134
- IND.ENG.CHEM.RES., vol. 44, 2005, pages 7596-7598, XP002463137

## Description

L'invention vise un procédé conjugué de synthèse d'une part de nitriles et/ou amines gras et d'autre part de carbonates de mono-alcools, à partir d'esters gras issus d'huiles naturelles.

L'évolution actuelle en matière d'environnement conduit dans les domaines de l'énergie et de la chimie à privilégier l'exploitation des matières premières naturelles provenant d'une source renouvelable. Ces contraintes environnementales imposent également d'éviter le transport et le stockage de matières premières dangereuses.

Un exemple de procédé industriel utilisant un acide gras comme matière première est celui de la fabrication de nitriles et/ou d'amines gras à partir d'acides gras extraits d'huiles végétales ou animales. Ce procédé est décrit dans l'encyclopédie Kirk-Othmer Vol 2, 4° Edition, page 411. L'amine grasse est obtenue en plusieurs étapes. La première étape consiste en une méthanolyse ou une hydrolyse d'une huile végétale ou d'une graisse animale produisant respectivement l'ester méthylique d'un acide gras ou un acide gras. L'ester méthylique de l'acide gras peut ensuite être hydrolysé pour former l'acide gras. Ensuite, l'acide gras est transformé en nitrile par réaction avec l'ammoniac, et finalement en amine par hydrogénation du nitrile ainsi obtenu.

La préparation des amines grasses à partir des acides gras via un nitrile date des années 1940. Le schéma réactionnel de la synthèse des nitriles peut se résumer de la façon suivante.

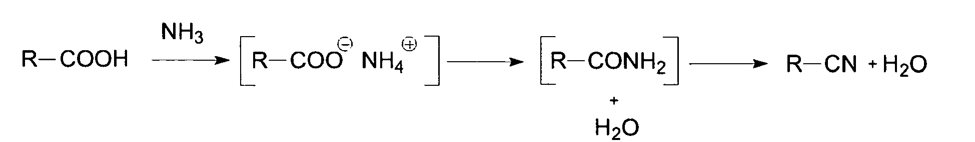

Il existe 2 types de procédés basés sur ce schéma réactionnel : un procédé batch en phase liquide qui est pratiqué par la société CECA et un procédé continu en phase vapeur.

Dans le procédé batch, on charge l'acide gras ou un mélange d'acides gras avec un catalyseur qui est généralement un oxyde métallique et le plus fréquemment l'oxyde de zinc. On porte le milieu réactionnel jusqu'à environ 150°C sous agitation, puis on commence à introduire l'ammoniac gazeux. Dans un premier temps, on forme un sel d'ammonium ou savon d'ammonium. La température du milieu réactionnel est ensuite portée aux alentours de 250°-300°C toujours sous introduction d'ammoniac. Le sel d'ammonium se transforme en amide avec libération d'une première molécule d'eau. Puis, dans un deuxième temps et avec l'aide du catalyseur, l'amide se transforme en nitrile avec formation d'une deuxième molécule d'eau. Cette eau formée est éliminée en continu du réacteur en entraînant l'ammoniac qui n'a pas réagi et un peu de chaînes grasses parmi les plus légères. Le passage par un déflegmateur rétrograde les composés gras vers le milieu réactionnel alors que les eaux ammoniacales sont envoyées vers un système de récupération de l'ammoniac qui peut être recyclé. La réaction est terminée quand il n'y a plus de fonction acide et que la teneur en amide est conforme aux spécifications.

Dans le procédé continu, la réaction a lieu en phase vapeur à des niveaux de température élevés et généralement sur un lit fixe d'alumine dopée ou non.

L'avantage du procédé batch est de conduire à des nitriles plus purs car ils sont généralement distillés, alors que ce n'est pas le cas en procédé continu. Par contre, le procédé en continu est plus avantageux lorsqu'il s'agit de nitriles à chaînes grasses insaturées ou poly-insaturées qui sont thermiquement plus sensibles que des composés à chaînes grasses saturées. En effet, dans ce procédé le temps de séjour est faible et l'indice d'iode, traduisant le nombre d'insaturations, est mieux conservé.

L'étape de synthèse des amines grasses consiste en une hydrogénation classique des nitriles gras. Les catalyseurs sont nombreux mais préférentiellement, on utilise le nickel de Raney ou le cobalt de Raney. Afin de favoriser la formation de l'amine primaire, on opère avec une pression partielle d'ammoniac, alors que dans le cas où l'on souhaiterait obtenir une amine secondaire on ne met pas de pression partielle d'ammoniac et on élimine si possible l'ammoniac formé en cours de réaction.

De nombreuses demandes de brevets décrivent les conditions pour effectuer la synthèse de nitriles d'acides gras ou d'amines grasses à partir d'acides gras ou d'esters d'acides gras. On peut citer par exemple le document JP 2000-16977 qui décrit la synthèse de nitriles à partir d'acides gras en utilisant une catalyse à l'oxyde de niobium à une température de 260°C dans le cas de l'acide stéarique. Le document JP 2000-7637 décrit la synthèse de nitriles à partir d'esters méthyliques d'acides gras linéaires ou ramifiés insaturés ou non, comportant de 6 à 22 atomes de carbone, et d'ammoniac à une température allant de 180° à 350°C en présence d'un catalyseur de type oxyde de niobium. Le brevet US 6,005,134, décrit un procédé de synthèse de nitriles aliphatiques à partir d'acides carboxyliques en C₆-C₂₂, d'alkyl esters d'acides carboxyliques en C₆-C₂₂ ou à partir de triglycérides, la réaction avec l'ammoniac étant réalisée en présence d'un catalyseur comprenant au moins du titane. Les résultats obtenus indiquent cependant des rendements inférieurs en partant d'esters ou de triglycérides, comparativement aux acides. Dans le brevet JP 10-195035, une catalyse à l'oxyde de zirconium dopé au fer conduit à des rendements excellents en nitrile, mais les auteurs mentionnent la présence d'amines légères liées à la formation de méthanol dans le cas des esters méthyliques.

Le procédé de synthèse des nitriles et/ou amines gras, opéré de manière industrielle à partir d'acides gras depuis plusieurs décennies donne globalement satisfaction. Cependant, il présente un certain nombre d'inconvénients. Le principal inconvénient est que sa mise en oeuvre est en pratique asservie à l'accès à une matière première spécifique, l'ammoniac en particulier, qui nécessite des précautions de stockage et d'utilisation coûteuses.

Lorsqu'un ester méthylique d'acide gras est utilisé comme source d'acide gras pour le procédé de synthèse des nitriles et/ou amines gras, le procédé produit des sous-produits qu'il faut valoriser séparément, notamment du méthanol lors de l'étape d'hydrolyse de l'ester. Ce méthanol est généralement traité pour être recyclé afin d'effectuer l'étape de méthanolyse d'une huile naturelle conduisant à l'ester méthylique d'acide gras.

Il existe plusieurs voies connues de synthèse du carbonate de mono-alcools tels que le diméthylcarbonate de formule CH₃O-CO-OCH₃ (DMC) ou le diéthylcarbonate (C₂H₅O-CO-OC₂H₅).

Les procédés industriels les plus anciens utilisent le phosgène comme matière première qui réagit avec l'alcool en milieu concentré de soude et cela en deux étapes successives, formation tout d'abord d'un chlorocarbonate puis du carbonate neutre. Ces procédés présentent des problèmes de sécurité en raison de la haute toxicité du phosgène ainsi que des déchets formés au cours du processus.

Les industriels se sont tournés alors vers des procédés de carbonylation oxydante ou des procédés de transestérification du carbonate d'éthylène.

Les procédés de carbonylation oxydante avec le couple oxydant CO + O₂ ont été développés soit en phase liquide, soit en phase gazeuse. Pour la phase liquide, on peut citer le procédé Enichem, brevets EP 534 545 et US 5,210,269, conduit en deux étapes en présence de chlorure de cuivre comme catalyseur. Pour la phase gazeuse, on peut citer le procédé UBE, brevet US 5,885,917, qui utilise NO comme agent actif avec un catalyseur au palladium, procédé comportant deux étapes avec le nitrite de méthyle comme produit intermédiaire.

Les dialkylcarbonates peuvent également être préparés par transestérification des carbonates d'alkylène avec l'alcool correspondant. De nombreuses sociétés ont travaillé sur ce type de réaction. On peut citer les brevets US 3,642,858 et US 3,803,201 de la société Dow qui décrivent un procédé de transestérification de carbonates d'alkylène avec des composés hydroxyles non tertiaires en présence de catalyseurs alcalins. La société Bayer a développé un procédé similaire en utilisant un catalyseur au thallium (brevet US 4,307,032). Texaco propose pour sa part des catalyseurs solubles à base de sels de zirconium, titane et/ou étain (brevet US 4,661,609) ou à base de composés trivalents soufre ou sélénium (brevet US 4,734,518). Enfin plus récemment la société Nippon Shokubai décrit l'utilisation de catalyseurs supportés d'oxydes de terres rares (US 5,430,170).

Dans la demande WO 2004/091778, Mitsubishi décrit une synthèse de dialkylcarbonates utilisant du CO₂ supercritique et un catalyseur solide acide hétérogène, pour faire réagir le CO₂ sur l'acétone diméthylacétal.

Parmi les méthodes de synthèse des dialkylcarbonates on relève les réactions de l'urée sur les mono-alcools.

Les réactions de carbonatation utilisant de l'urée ont déjà été décrites pour des mono-alcools par exemple par « Shaikh et Sivaram, Organic Carbonates, Chem rev. 1996, 96, 951 », « Ball et al., Synthesis of carbonates and polycarbonates by reaction with urea with hydroxy compounds, Mol. Chem. 1984, vol.1, p. 95-108 ».

M. Wang, et al. dans « Catalysis Communication 7 (2006) 6-10 » décrivent la synthèse de diméthylcarbonate à partir de méthanol et d'urée sur des solides basiques. Cette réaction comporte deux étapes avec la formation intermédiaire du méthylcarbamate lui-même transformé en diméthylcarbonate. Malheureusement, si la première étape permet l'obtention de bons résultats la seconde est beaucoup plus délicate avec des réactions secondaires, et dans ce cas les sélectivités de la réaction globale restent faibles.

Dans un brevet US 5,902,894, Catalytic Distillation Technologies décrit un procédé de synthèse du DMC utilisant des solvants organiques à haut point d'ébullition et donneurs d'électron ; par exemple le diméthyléther du triéthylèneglycol, associé à un catalyseur à l'étain permettait d'obtenir de bons rendements et de bonnes sélectivités en DMC en pratiquant une extraction par distillation en continu du DMC formé. De manière analogue, Exxon, dans la demande de brevet WO 95/17369, minimise la formation des sous-produits par une distillation extractive du dialkylcarbonate.

Enfin, plus récemment, la demande de brevet chinois CN 1431190 (Shanxi Institute) décrit un procédé de synthèse de DMC à partir de méthanol et d'urée, utilisant des catalyseurs à base d'étain et d'un co-catalyseur.

Dans le procédé de synthèse des nitriles et/ou amines gras, il est nécessaire d'importer et de stocker des quantités importantes d'ammoniac, ce qui représente outre le problème du transport, un risque majeur en cas de fuite, non seulement pour le personnel mais aussi pour le voisinage. Ce stockage impose par conséquent des contraintes importantes au site exploitant ce type de procédé.

Le problème à résoudre est de limiter ces risques environnementaux tout en maintenant les performances du procédé ou même mieux en les améliorant.

L'invention vise donc un procédé conjugué de production de nitriles et/ou amines gras et de carbonates de mono-alcools à partir d'un ester d'acide gras saturé ou insaturé issu d'huiles naturelles, dans lequel le même ester d'acide gras sert de matière première pour la synthèse de nitriles et/ou amines d'une part et de carbonates de mono-alcools d'autre part. La synthèse des carbonates de mono-alcool est réalisée par action de l'urée sur le mono-alcool, l'ammoniac produit par cette réaction est utilisé comme réactif pour l'étape d'ammoniation lors de la synthèse des nitriles et/ou amines gras.

L'invention vise un procédé conjugué de production de nitriles et/ou amines gras et de carbonates de mono-alcool de type dialkylcarbonates de formule R₁O-CO-OR₁ dans laquelle R₁ comporte de 1 à 4 atomes de carbone, à partir d'un ester d'un acide gras insaturé ou non issu d'huiles naturelles comportant les zones suivantes :
I) zone de synthèse d'un nitrile/amine comportant les étapes suivantes :
   a) transformation optionnelle tout d'abord par hydrolyse de l'ester de l'acide gras issu d'une huile d'origine naturelle, de formule R-COOR₁ dans laquelle R est un radical alkyle saturé ou insaturé comportant de 7 à 21 atomes de carbone, de préférence de 11 à 17 atomes de carbone, R₁ un radical alkyle comprenant de 1 à 4 atomes de carbone, en acide gras de formule R-COOH et produisant simultanément l'alcool R₁OH qui est récupéré et adressé à la zone III, suivie en b) d'une ammoniation avec de l'ammoniac de l'acide gras issu de l'étape a) et/ou de l'ester de l'acide gras de formule R-COOR₁ en nitrile produisant respectivement un mélange ammoniac/eau et/ou un mélange alcool/ammoniac/eau qui sont envoyés à la zone IV, puis suivie en c) d'une hydrogénation du nitrile issu de l'étape b), éventuellement en présence d'ammoniac, transformant le nitrile en amine correspondante,
II) zone de synthèse d'un carbonate de mono-alcool par réaction de l'urée sur l'alcool R₁OH, la réaction dudit alcool avec l'urée produisant également de l'ammoniac,
III) zone de récupération et de purification de l'alcool R₁OH qui sert d'alimentation pour la zone II,
IV) zone de récupération de l'ammoniac issue de la zone II ainsi que celle issue de la zone Ib où la réaction est réalisée avec un excès d'ammoniac, et éventuellement celle issue de la zone Ic, pour servir d'alimentation pour l'ammoniation de l'étape Ib et éventuellement l'hydrogénation de l'étape Ic de la zone I.

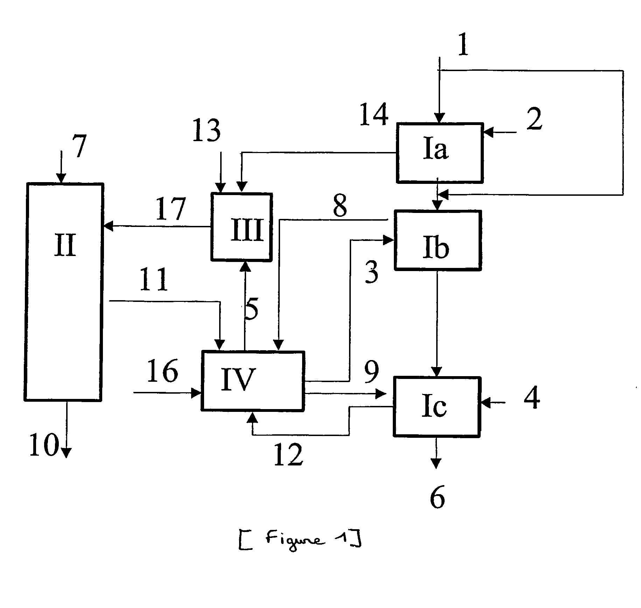

Le procédé de l'invention est décrit en référence au schéma simplifié annexé en figure 1 en prenant pour exemple la synthèse du DMC à partir de l'ester méthylique d'un acide gras qui peut être obtenu directement par méthanolyse d'une huile végétale. Il va de soi qu'exactement le même processus peut être mis en oeuvre avec des esters d'acide gras tels que les esters éthylique, propylique, isopropylique ou butylique permettant la synthèse à côté du nitrile / amine, de diéthylcarbonate, dipropylcarbonate, di(iso)propylcarbonate, diterbutylcarbonate ou di(iso)butylcarbonate. Dans le cas où l'ester à traiter n'est pas disponible sur le marché, il est naturellement possible de procéder au préalable à l'estérification de l'acide gras issu de l'huile naturelle.

La zone I est alimentée par la ligne 1 en un ester d'acide gras, acide provenant d'une huile naturelle, de formule R-COOR₁ dans laquelle R₁ est un radical méthyle, éthyle, propyle, butyle ou isopropyle, et R est un radical alkyle saturé ou insaturé comportant de 7 à 21 atomes de carbone, de préférence de 11 à 17 atomes de carbone. Dans la zone la, l'ester d'acide gras est transformé en acide gras R-COOH par hydrolyse avec de l'eau introduite par la ligne 2, produisant simultanément l'alcool R₁OH qui est récupéré et adressé à la zone III par la ligne 14. L'acide gras issu de la zone la, et/ou l'ester d'acide gras introduit par la ligne 1 sont introduits dans la zone Ib où ils sont soumis à une réaction d'ammoniation avec de l'ammoniac en excès introduit par la ligne 3, produisant respectivement un mélange ammoniac/eau et/ou un mélange alcool/ammoniac/eau qui sont envoyés à la zone IV par la ligne 8 pour purification, selon les processus réactionnels suivants :

R-COOH + NH₃ → R-CN + 2H₂O

R-COOR₁ + NH₃ **→** R-CN + H₂O + R₁OH

Le nitrile gras formé en zone Ib est ensuite transféré en zone Ic où il est soumis à une hydrogénation avec de l'hydrogène introduit par la ligne 4, et éventuellement en présence d'ammoniac introduit par la ligne 9, afin d'obtenir l'amine grasse primaire selon le processus réactionnel suivant :

R-CN + 2H₂ (NH₃) → R-CH₂-NH₂

L'ammoniac est recyclé par la ligne 12 et envoyé à la section IV pour purification et l'amine grasse est extraite de la zone I par la ligne 6.

L'alcool R₁OH issu de la zone la est adressé par la ligne 14 à la zone III ainsi que l'alcool issu de la zone IV de purification est adressé par la ligne 5 à la zone III. Une ligne d'appoint 13 peut en outre amener de l'alcool directement dans la zone III. Cet alcool, après purification, sert d'alimentation par la ligne 17 de la zone II pour la réaction avec l'urée introduit par la ligne 7 pour former le carbonate de l'alcool R₁OH selon le processus réactionnel suivant :

2R₁OH + NH₂-CO-NH₂ → R₁O-CO-OR₁ + 2 NH₃

Le carbonate de l'alcool R₁OH synthétisé est extrait de la zone II par la ligne 10 et l'ammoniac formé est adressé à la zone IV par la ligne 11.

La zone IV comporte outre les lignes d'alimentation d'ammoniac 11 et 12 et la ligne d'alimentation d'un mélange alcool/ammoniac/eau et/ou ammoniac/eau 8, une ligne d'appoint 16 permettant notamment de fournir l'ammoniac nécessaire au démarrage de l'unité et à sa régulation en cas de variation de la production d'ammoniac au niveau de la zone II. L'ammoniac issu de la zone Ib est dirigé vers la zone IV par la ligne 8, l'alcool éventuellement présent dans ce flux est séparé et envoyé avec l'eau produite en zone Ib vers la section III par la ligne 5.

Le procédé est particulièrement bien adapté à la synthèse d'amine grasse et de diméthylcarbonate à partir d'ester méthylique produit par méthanolyse du triglycéride. Les différentes réactions seront les suivantes :
Zone la : R-COOCH₃ + H2O → R-COOH + CH₃OH
Zone Ib : R-COOH + NH₃ → R-CN + 2 H₂O
Zone Ic : R-CN + 2 H₂ (NH₃) → R-CH₂-NH₂
Zone II : 2 CH₃OH + NH₂-CO-NH₂ → CH₃O-CO-OCH₃ + 2 NH₃

Les conditions de mise en oeuvre des différentes étapes des zones I et II, sont connues de l'homme de l'art. On peut cependant préciser que :
- la réaction d'hydrolyse de l'étape a) de la zone I est réalisée par exemple à la température ambiante, ou à une température plus élevée.
- l'ammoniation de l'étape b) de la zone I conduisant au nitrile est réalisée à une température de 150° à 400°C, et de préférence à une température voisine de 300°C.
- l'hydrogénation de l'étape c) dans la zone I conduisant à une amine est réalisée à une température allant de 80° à 170°C, de préférence en présence d'ammoniac pour former l'amine primaire.
- la conversion de l'alcool R₁OH en dialkylcarbonate réalisée au niveau de la zone II est comme cela a été indiqué précédemment une réaction connue depuis plusieurs années. Pour la conduite du procédé, la solution retenue est la réaction généralement nommée transestérification de l'alcool R₁OH par l'urée. Cette réaction est conduite en présence de catalyseurs connus en soi, par exemple ceux décrits dans les brevets US 6,495,703, oxyde de zinc, ou EP 0 955 298, sulfates métalliques ou organométalliques, et de préférence des catalyseurs à base d'étain tels que ceux décrits dans les brevets US 5,902,894, WO Application 95/17369 ou CN 1431190.

Les zones III et IV sont des zones de stockage et de purification de l'alcool R₁OH d'une part, et de l'ammoniac d'autre part, qui sont réalisées selon des techniques bien connues de l'homme de l'art. L'alcool est par exemple séparé de l'eau par distillation et par séchage sur des tamis moléculaires. L'ammoniac est utilisé sous forme gazeuse.

On peut observer que le procédé peut être considéré comme quasi autonome en bilan matière ce qui est exceptionnel. En effet, l'étape Ib produit une quantité de mono-alcool qui est transférée, après purification, dans la zone II pour réagir avec l'urée dans le rapport de 2 moles d'alcool pour une mole d'urée. Cette réaction de synthèse du dialkylcarbonate produit elle-même deux moles d'ammoniac, quantité juste nécessaire à l'ammoniation de l'étape Ib de la zone I. Outre l'économie entraînée, la limitation du stockage d'ammoniac sur le site est bénéfique sur le plan environnemental.

## Revendications

1. Procédé conjugué de production de nitriles et/ou amines gras et de carbonates de mono-alcool de type dialkylcarbonates de formule R₁O-CO-OR₁ dans laquelle R₁ comporte de 1 à 4 atomes de carbone, à partir d'un ester d'un acide gras insaturé ou non issu d'huiles naturelles, comportant les zones suivantes :
I) zone de synthèse d'un nitrile/amine comportant les étapes suivantes : a) transformation optionnelle tout d'abord par hydrolyse de l'ester de l'acide gras issu d'une huile d'origine naturelle, de formule R-COOR₁ dans laquelle R est un radical alkyle saturé ou insaturé comportant de 7 à 21 atomes de carbone, R₁ un radical alkyle comprenant de 1 à 4 atomes de carbone, en acide gras de formule R-COOH, avec introduction d'eau, et produisant simultanément l'alcool R₁OH qui est récupéré et adressé à la zone III, suivie en b) d'une ammoniation avec de l'ammoniac de l'acide gras issu de l'étape a) et/ou de l'ester de l'acide gras de formule R-COOR₁ en nitrile produisant respectivement un mélange ammoniac/eau et/ou un mélange alcool/ammoniac/eau qui sont envoyés à la zone IV, puis suivie en c) d'une hydrogénation du nitrile issu de l'étape b), à l'aide d'hydrogène 4, éventuellement en présence d'ammoniac, transformant le nitrile en amine correspondante, avec recyclage éventuel de l'ammoniac à la zone IV,
II) zone de synthèse d'un carbonate de mono-alcool par réaction de l'urée sur l'alcool R₁OH, réaction dudit l'alcool avec l'urée produisant également de l'ammoniac, avec extraction du carbonate de mono-alcool et de l'ammoniac formé adressé à la zone IV,
III) zone de récupération et de purification de l'alcool R₁OH issu de la zone la ou issu de la zone IV, qui sert d'alimentation pour la zone II,
IV) zone de récupération de l'ammoniac issue de la zone II ainsi que celle issue de la zone Ib où la réaction est réalisée avec un excès d'ammoniac, et éventuellement celle issue de la zone Ic, pour servir d'alimentation pour l'ammoniation de l'étape Ib, et éventuellement pour l'hydrogénation de l'étape Ic de la zone I.

2. Procédé selon la revendication 1 **caractérisé en ce que** l'ester de la charge répond à la formule R-COOCH₃ qui est soumis lors de l'étape la à une hydrolyse pour former à la sortie des étapes de la zone I une amine grasse de formule R-CH₂-NH₂, et le diméthylcarbonate à la sortie de la zone II.

## Claims

1. Joint process for the production of fatty nitriles and/or amines and of monoalcohol carbonates of dialkyl carbonate type of formula R₁O-CO-OR₁, in which R₁ comprises from 1 to 4 carbon atoms, from an ester of a saturated or unsaturated fatty acid resulting from natural oils, comprising the following zones:
I)zone of synthesis of a nitrile/amine comprising the following stages: a) optional conversion first of all by hydrolysis of the fatty acid ester resulting from an oil of natural origin, of formula R-COOR₁, in which R is a saturated or unsaturated alkyl radical comprising from 7 to 21 carbon atoms and R₁ is an alkyl radical comprising from 1 to 4 carbon atoms, to give a fatty acid of formula R-COOH, with introduction of water, with simultaneous production of the alcohol R₁OH, which is recovered and sent to the zone III, followed, in b), by an ammoniation with ammonia of the fatty acid resulting from stage a) and/or of the fatty acid ester of formula R-COOR₁ to give a nitrile, respectively producing an ammonia/water mixture and/or an alcohol/ammonia/water mixture, which are conveyed to the zone IV, then followed, in c), by a hydrogenation of the nitrile resulting from stage b) using hydrogen, optionally in the presence of ammonia, which converts the nitrile to the corresponding amine, with optional recycling of the ammonia to the zone IV,
II) zone of synthesis of a monoalcohol carbonate by reaction of urea with the alcohol R₁OH, the reaction of said alcohol with urea also producing ammonia, with extraction of the monoalcohol carbonate and of the ammonia formed sent to the zone IV,
III) zone of recovery and purification of the alcohol R₁OH resulting from the zone Ia or resulting from the zone IV, which acts as feed for the zone II,
IV) zone of recovery of the ammonia resulting from the zone II and that resulting from the zone Ib, where the reaction is carried out with an excess of ammonia, and optionally that resulting from the zone Ic, to act as feed for the ammoniation of stage Ib and optionally for the hydrogenation of stage Ic of the zone I.

2. Process according to Claim 1, **characterized in that** the ester of the charge corresponds to the formula R-COOCH₃, which is subjected, during stage Ia, to a hydrolysis in order to form, at the outlet of the stages of the zone I, a fatty amine of formula R-CH₂-NH₂ and, at the outlet of the zone II, dimethyl carbonate.

## Patentansprüche

1. Konjugiertes Verfahren zur Herstellung von Fettnitrilen und/oder -aminen und Monoalkoholcarbonaten vom Dialkylcarbonat-Typ der Formel R₁O-CO-OR₁, worin R₁ 1 bis 4 Kohlenstoffatome enthält, ausgehend von einem aus natürlichen Ölen stammenden Ester einer ungesättigten oder gesättigten Fettsäure mit den folgenden Zonen:
I) Zone zur Synthese eines Nitrils/Amins mit den folgenden Schritten: a) gegebenenfalls zunächst Umwandlung des aus einem Öl natürlichen Ursprungs stammenden Fettsäureesters der Formel R-COOR₁, worin R für einen gesättigten oder ungesättigten Alkylrest mit 7 bis 21 Kohlenstoffatomen steht und R₁ für einen Alkylrest mit 1 bis 4 Kohlenstoffatomen steht, in eine Fettsäure der Formel R-COOH durch Hydrolyse mit Eintragen von Wasser und gleichzeitiger Herstellung des Alkohols R₁OH, der wiedergewinnen und der Zone III zugeführt wird, in b) gefolgt von einer Ammonierung der Fettsäure aus Schritt a) und/oder des Fettsäureesters der Formel R-COOR₁ mit Ammoniak unter Herstellung einer Mischung von Ammoniak und Wasser und/oder einer Mischung von Alkohol, Ammoniak und Wasser, die zur Zone IV befördert werden, zu einem Nitril, dann in c) gefolgt von einer Hydrierung des Nitrils aus Schritt b) mit Hilfe von Wasserstoff, gegebenenfalls in Gegenwart von Ammoniak, wodurch das Nitril in das entsprechende Amin umgewandelt wird, mit eventueller Rezyklierung des Ammoniaks in Zone IV,
II) Zone zur Synthese eines Monoalkoholcarbonats durch Reaktion von Harnstoff mit dem Alkohol R₁OH, wobei bei der Reaktion des Alkohols mit dem Harnstoff auch Ammoniak anfällt, mit Extraktion des Monoalkoholcarbonats und des der Zone IV zugeführten Ammoniaks,
III) Zone zur Rückgewinnung und Reinigung des Alkohols R₁OH aus der Zone Ia oder der Zone IV, der als Einsatz für die Zone II dient,
IV) Zone zur Rückgewinnung des Ammoniaks aus Zone II sowie des Ammoniaks aus Zone Ib, in der die Reaktion mit einem Überschuss von Ammoniak durchgeführt wird, und gegebenenfalls des Ammoniaks aus Zone Ic als Einsatz für die Ammonisierung von Schritt Ib und gegebenenfalls für die Hydrierung von Schritt Ic der Zone I.

2. Verfahren nach Anspruch 1, **dadurch gekennzeichnet, dass** der Ester der Beschickung der Formel R-COOCH₃ entspricht, der in Schritt Ia einer Hydrolyse unterworfen wird, um am Ausgang der Schritte der Zone I ein Fettamin der Formel R-CH₂-NH₂ und am Ausgang von Zone II Dimethylcarbonat zu bilden.
